# EUROPEAN PATENT APPLICATION

(11) **EP 2 677 320 A1**
(43) Date of publication of application: **25.12.2013**
(21) Application number: 12305713.5
(22) Date of filing: 21.06.2012
(51) Int. Cl.: G01N 33/80

(54) **Methods and kit for assessing the quality of red blood cells generated in vitro**

(71) Applicant: Université Paris 6 Pierre et Marie Curie UPMC, 75005 Paris (FR); Etablissement Français Du Sang (EFS), 93218 La Plaine Saint Denis (FR)
(72) Inventor: Giarratana, Marie-Catherine, 93400 Saint Ouen (FR); Douay, Luc, 75007 Paris (FR)
(74) Representative: Domenego, Bertrand

(57) **Abstract**

The present invention relates to a method for assessing the quality of batches of red blood cells generated *in vitro,* and to a method for evaluating the suitability of batches of red blood cells generated *in vitro* for long-term storage. The invention also concerns kits that are useful in the above methods.

## Description

### FIELD OF THE INVENTION

The present invention relates to a method for assessing the quality of batches of red blood cells generated *in vitro,* and to a method for evaluating the suitability of batches of red blood cells generated *in vitro* for long-term storage. The invention also concerns kits that are useful in the above methods.

### BACKGROUND

For many years, blood transfusion has been confronted with constant difficulties in obtaining supplies, linked to the increasing need for blood products and a shortage of donors.

It has become an urgent necessity to find alternatives to the classical blood donations. Hence, attempts to generate erythroid cells from cells of diverse origins make good sense. Although the use of fetal cells or human embryonic or induced pluripotent cells still requires adjustments notably in terms of the level of enucleation, the mass production *in vitro* of functional enucleated cells from hematopoietic stem cells isolated from cord blood or by leukapheresis marked an important advance (see Kim HO et Baek EJ., Tissue Eng. Part A, 2011; Migliaccio et al., Curr. Opin. Hematol., 16: 259-68, 2009; Baek et al., Transfusion;48: 2235-45, 2008; Fujimi et al. Int. J. Hematol.;87: 339-50, 2008; Vlaski et al., Exp. Hematol., 37: 573-84, 2009; Boehm et al., J. Biotechnol.,144: 127-34, 2009; Giarratana et al., Nat. Biotechnol., 23: 69-74, 2005). In this area, it has recently been shown that red blood cells generated *in vitro* (also called cultured red blood cells, abbreviated as cRBC), which have characteristics of reticulocytes, survive in the blood circulation after injection into humans just as transfused cells do.

However, the *in vitro* production of functional cRBC is the culmination of a multiparametric technological procedure, which makes it difficult to control the products. In particular, in a cell population of functional cRBCs, a certain percentage of cells may differ from native red blood cells. For instance, they may be damaged and unsuitable for a long-term storage.

This is a problem because cRBC are mainly intended to be stored for extended periods of time in blood banks in transfusion centers and hospitals before being infused to patients in need.

Qualitative evaluation of cultured RBC (cRBC) would contribute to biotechnological progress because transfusion efficacy depends in large part on the quality of the injected cells.

However, to date no method has been developed to evaluate the quality cRBCs and their capacity to be stored for a long period of time.

Thus, it is desirable to develop methods for assessing the quality of a batch of red blood cells, in particular to determine if a batch of red blood cells generated *in vitro* is suitable for being stored for a long period of time in blood banks.

### DESCRIPTION OF THE INVENTION

The inventors observed that the suitability of cRBCs for long-term storage at 4°C may strongly vary from one batch of cRBCs to another. They found that after three weeks of storage at 4°C, the cell loss in a batch rangedfrom 4 to 44%.

In an attempt to develop a method for evaluating the quality of cRBCs at the end of the *in vitro* production process, in particular for evaluating the capacity of cRBCs to be stored in blood banks, the inventors monitored the markers classically used to evaluate native red blood cells (i.e. cell viability, expression of surface markers CD47, externalisation of phosphatidylserine). However, they found no correlation between these markers and the ability of cRBC batches to be stored has been found.

Seeking a reliable marker useful for sorting cRBC batches according to their ability to be stored, the inventors unexpectedly found that an erythrophagocytosis assay allows cRBC batches suitable for being stored during a long period of time to be distinguished from cRBC batches unsuitable for being stored during a long period of time. In particular, they observed a relationship between the cell yield (or the cell loss) after 3 weeks of storage and the phagocytic index at time t0 of the conservation test carried out with cRBCs.

Thus, the inventors have demonstrated for the first time that testing phagocytosis of cRBC at the end of the production line allows sorting of batches that are suitable for being stored for a long period of time and subsequently transfused to patients.

Interestingly, the inventors also found that an erythrophagocytosis assay carried out with non professional phagocytes, more particularly with the murine fibroblast cell line MS-5, is clearly more sensitive than an assay performed with human macrophages (professional phagocytes).

### Summary of the invention

Therefore, the present invention relates to methods for assessing the quality of a batch of red blood cells generated *in vitro* (cRBCs) and for evaluating its capacity to be stored for a long period of time.

The invention further provides kits that are useful in the above methods, as described below.

### Detailed description of the invention

These and other objects, features and advantages of the invention will be disclosed in the following detailed description.

The present invention relates to a method for assessing the quality of a batch of red blood cells generated *in vitro* (cRBCs), which method comprises the following steps:
a) bringing into contact with phagocytes a sample of cRBCs from the batch to be tested;
b) determining the percentage of the cRBCs which are phagocytosed (erythrophagocytosis quantification);
c) optionally, deducing from the result of step b) the quality of the batch of cRBCs. The invention also concerns a method for evaluating the capacity of a batch of red blood cells generated *in vitro* (cRBCs) to be stored for a long period of time, which method comprises the following steps:
   a) bringing into contact with phagocytes a sample of cRBCs from the batch to be tested;
   b) determining the percentage of the cRBCs which are phagocytosed (erythrophagocytosis quantification);
   c) optionally, deducing from the result of step b) the capacity of said batch cRBCs to be stored for a long period of time.

The inventors have found a relationship between the cell recovery of cRBCs in a batch after long-term storage and the percentage of erythrophagocytosis at the end of the *in vitro* production process, i.e. before storage. The higher the percentage of erythrophagocytosis, the poorer the quality of the tested batch of red blood cells generated *in vitro.*

Hence, the measurement of no or low erythrophagocytosis in step c) of the methods is indicative of a batch of red blood cells generated *in vitro* of good quality, said batch being suitable for long-term storage, whereas the measurement of high percentage of erythrophagocytosis in step c) of the methods is indicative of a batch of red blood cells generated *in vitro* of a poor quality which is not suitable for long-term storage.

In particular, a batch of cRBCs is considered as being of good quality when at least about 70%, preferably at least about 75%, still preferably at least about 80%, 85%, advantageously 90%, 91%, 92%, 93%, 94%, and ideally 95%, 96%, 97%, 98%, 99% or 100% of the cRBCs of the sample to be tested are not phagocytosed.

A batch of cRBCs is considered as being suitable for long-term storage when the batch of cRBCs is of good quality.

Step (a) is conducted for a time sufficient to allow the cRBCs of poor quality (i.e. cells with damaged membranes) to be phagocytosed and can be easily determined by one of ordinary skill in the art. Typically, step (a) is carried out for 1 to 5 hours, preferably two and a half hours to three and a half hours.

Preferably, the ratio [number of cRBCs / number of phagocytes] is from 10/1 to 1/80, more preferably from 20/1 to 40/1. The sample to be tested may, for example, consist of 10⁵ to 10⁸ cRBCs.

In a preferred embodiment, before step (b) is a step (a') consisting of removing the non-phagocytosed cRBCs. For instance, non-phagocytosed cRBCs are lysed (e.g. by washing the mix of cRBCs and phagocytes of step (a) in distilled water for 1 minute, followed by restoration of isotonicity to avoid damaging phagocytes), then cell debris and haemoglobin are removed (e.g. by washing in a buffer solution, for example PBS).

Preferably, the methods are carried out at about 37°C for step (a) and at room temperature for step a') consisting of removing the non-phagocytosed cRBC.

In a preferred embodiment of the methods according to the invention, the cRBCs of the sample to be tested are artificially labelled *in vitro* to make the quantification of the phagocytosis of the cRBCs easier.

The labelling of cRBCs, for example, may be carried out with fluorochrome markers as described by Bratosin et al. (Cytometry, 30: 269-274, 1997; labelling with PKH-26, a non-toxic lipophilic dye), Fendel et al. (Cytometry Part A 71 A: 258-264, 2007; labelling with carboxyfluorescein-diacetate-succinimidyl ester, abbreviated as "CFDA-SE).

Preferably, the cRBCs are labelled with CFDA-SE.

When the cRBCs are labelled with a fluorochrome marker, the quantification of step (b) may be done by flow cytometric quantification of the number of phagocytosed fluorescent cRBCs (quatification of erythrophagocytosis).

The cRBCs may also be labelled with radioactive markers, for instance 51Cr (see Kuster and Schauer, Hoppe Seylers Z Physiol. Chem., 362: 1507-1514, 1981), ¹⁴C-cyanate (see Bussolino et al., Br. J. Haematol., 66: 271-274, 1987,) (111)-Indium or (99m)-Technetium (see Davey RJ, Transf. Med. Rev, 2:151-160, 1988).

The markers used must not be toxic for the cRBCs and the phacocytes.

Alternatively, the red blood cells generated *in vitro* are not labelled and the percentage of phagocytosed cRBCs is determined by microscopic counting of cRBCs within phagocytes. Preferably, after step (a) and before counting (step (b), the cells are stained with an appropriate biological dye, for instance the May Grunwald Giemsa dye, a mixture of eosin and methylene blue (Wright's stain), a mixture of hematoxylin and aqueous eosin solution (hematoxylin-eosin stain), for easier counting.

It is to be noted that the batches of cRBCs of good quality, and thus suitable for long-term storage, obtainable by (i.e. sorted by) the methods of the invention are also part of the present invention.

The invention further discloses a kit for assessing the quality of a batch of red blood cells generated *in vitro* (cRBCs), and/or for evaluating the capacity of said batch to be stored for a long period of time. The kit of the invention comprises the following means:
i) phagocytes;
ii) at least a marker chosen from fluorochrome markers and radioactive markers as described above, or at least a biological dye as described above;
iii) a positive control sample consisting of red blood cells, the red blood cells of which are characterized in that all of them (or at least almost all of them) would be phagocytosed if they were contacted with phagocytes.

The red blood cells are native red blood cells or red blood cells generated *in vitro.*

Preferably, the red blood cells (native red blood cells or red blood cells generated *in vitro*) (iii) are chosen from the group consisting of:
(a) aged red blood cells (e.g. red blood cells which have been stored 2 to 6 weeks at 4°C, most of them being damaged senescent cells that are recognized by phagocytes);
(b) heat-denatured red blood cells (e.g. prepared according to the protocol as described in the experimental part of the present application);
(c) desialized-red blood cells (e.g. prepared by treatment with neuraminidase according to the protocol as described in the experimental part of the present application);
(d) red blood cells treated with glutaraldehyde (these cells are recognized by phagocytes and phagocytosed).

Advantageously, the red blood cells (iii), in particular cells (a), (b) and (c), were treated with glutaraldehyde (employed alone or mixed with paraformaldehyde), and therefore are provided in glutaraldehyde solution or in glutaraldehyde / formaldehyde mix solution. In the context of the invention, the glutaraldehyde is used as a fixative / preservative for long-term storage.

In a preferred embodiment of the kit, the red blood cells (iii) are labeled with the same marker (fluorochrome markers or radioactive markers) as the one comprises in the kit. More preferably, the marker is the fluorochrome CFDA-SE.

In another preferred embodiment of the kit, the red blood cells (iii) are stained with the same dye as that is contained in the kit.

In a particularly preferred embodiment of the kit, the phagocytes are the murine fibroblast cell line MS-5.

The kit may further comprise, in addition to (i) to (iii), a set of calibration solutions (iv) which have been prepared with the positive control, the percentage of erythrophagocytosis of each calibration solutions is known.

The kit may also includes instructions (v) for the use of said kit in assessing the quality of a batch of red blood cells generated *in vitro,* and/or in evaluating the capacity of said batch to be stored for a long period of time, said instruction comprising a standard calibration curve which shows the relationship between the percentage of erythrophagocytosis measured in a cRBC sample and the quality of said sample (i.e. the capacity of the cRBCs of the sample to be stored for a long period of time).

Such a kit may for example comprise (i) (ii), (iii), and (iv), (i) (ii), (iii), and (v) or (i) (ii), (iii), (iv) and (v).

The kit of the invention may also include reagents such as washing buffers.

The means of the kit may be present, e.g., in vials or microtiter plates.

The kit may for example be stored between -20 and -80°C

### Definitions

The expression "the quality of a batch of red blood cells generated *in vitro*" refers to a batch of red blood cells generated *in vitro* wherein at least 70%, preferably at least 75%; and in order of preference at least 80, 85, 90%, 91%, 92%, 93%, 94%, 95%, 96%, 97%, 98%, 99% or 100%, of the red blood cells generated *in vitro* are not damaged and are still alive and functional after several weeks of storage.

The expression "long-term storage" and "stored for a long period of time" are used in the present application interchangeably, and refers to storage of the cRBCs lasting one to several weeks, preferably at least 2 weeks, still preferably at least 3 weeks, still preferably at least 4 weeks, typically at a temperature of 2 to 6°C, preferably at 4°C.

By "red blood cells generated *in vitro*" (also known as "cultured red blood cells", abbreviated as "cRBC") it is intended to mean erythroid cells generated from all types of stem cells (totipotent and pluripotent stem cells), progenitors or precursors cells which are able to differentiate into erythroid cells. In a particular embodiment, the cRBC are engineered for drug delivery or ectopic molecule expression.

Preferably, the red blood cells generated *in vitro* are of human origin (i.e. the stem cells, progenitors or precursors used for generating them come from human being).

The stem cells may be, for example, foetal stem cells, embryonic stem cells, or adult stem cells. In a preferred embodiment, the stem cells do not come from foetal or embryonic stem cells.

Preferably, the stem cells are hematopoietic stem cells and/or CD34 positive cells. In an embodiment, the CD34 positive cells used to generate cRBC may be prepared from any biological sample from an adult subject, such as blood, e.g. peripheral blood, bone marrow, cord blood or fetal liver. For instance blood samples may be normal Peripheral Blood mobilized with G-CSF [Leukapheresis (LK)] or not (PB) as described by Anderlini P et al., Transfusion, 39: 555-560, 1999.

The haematopoietic stem cells (CD34+ cells) and other cells that are able to be differentiated into erythroid cells can be isolated using commercially available antibodies that bind to haematopoietic stem cell surface antigens, e.g. CD34, using methods known to those of skill in the art.

Methods for isolating embryonic and adult stem cells are well known to those skilled in the art, and are for example disclosed by Thomson JA et al., (Science, 282: 1145-1147, 1998); Van Epps et al., (Blood Cells, 20: 411-423, 1994); Lebkoowski JS et al., Cancer, 7 (S2): S83-S93, 2001); Gordon PR et al., (Bone marrow transplant, 31: 17-22, 2002).

The stem cells may also be induced pluripotent stem cells (hereafter abbreviated "iPS"), i.e. a population of cells with characteristics reminiscent of embryonic stem cells which is generated from somatic tissues through nuclear reprogramming via the ectopic expression of genes related to pluripotency. Processes for generating iPS cells are for instance described by Takahashi et al. (Cell, 131: 861-872, 2007), Yu et al. (Science, 318: 1917-1920, 2007) and Okita et al. (Nature, 448: 313-317, 2007).

Methods for *in vitro* generation of red blood cells are well known to those skilled in the art, and are for example disclosed by Kim HO et Baek EJ. (Tissue Eng. Part A, 2011), Migliaccio et al. (Curr. Opin. Hematol., 16: 259-68, 2009), Baek et al. (Transfusion;48: 2235-45, 2008), Fujimi et al. (Int. J. Hematol.;87: 339-50, 2008), Vlaski et al. (Exp. Hematol., 37: 573-84, 2009), Boehm et al. (J. Biotechnol., 144: 127-34, 2009), Van den Akker E (Haematologica, 95: 1594-1598, 2010); Tirelli V et al., Stem Cells Int., 602483, 2011), and Giarratana et al. (Nat. Biotechnol., 23: 69-74, 2005). A preferred method of generating red blood cells is the protocol previously described by the present inventors that consists of a massive expansion of CD34+ stem cells/progenitors followed by their complete differentiation into perfectly functional mature RBCs (see US application N°11/597,509 and article by Giarratana et al., Nat. Biotech., 23(1): 69-74, 2005). Briefly, for example, the CD34+ cells are cultured in the presence of IMDM supplemented with human plasma and with sequential addition of SCF, IL-3 and Epo. The cells are fed twice a week with fresh medium. After about 18 days, a majority of the cells are reticulocytes (monitoring by measuring CD71 expression and by methylene blue staining). The cell suspensions are then purified by passage through a deleukocyting filter to eliminate the expelled nuclei and residual erythroblasts. The purity of the cRBC samples generally ranges from 99.1 to 99.5% after filtration.

By "native red blood cells" it is intended to mean erythroid cells from peripheral blood obtained directly from a subject (i.e. red blood cells generated *in vivo).*

In the context of the present invention the term "red blood cells" refers to all types of erythroid cells, in particular to mature erythrocytes or reticulocytes (immature red blood cells), or a mix thereof (both enucleate red cells).

The term "phagocytes" refers to cells which have the ability to engulf a solid particle or cell (here a red blood cell) to form an internal vesicle (a phagosome) that comprises said particle or cell).

In the context of the invention, the phagocytes are chosen from professional phagocytes (for instance polynuclear granulocytes, monocytes, dendritic cells and macrophage) and non-professional phagocytes (e.g. fibroblastic cells and epithelial cells).

Preferably, the phagocytes are non-professional phagocytes, and more preferably the non professional phagocytes are the murine fibroblast cell line MS-5 (also called murine stromal cell line MS-5, cells which has been established by Itoh et al. (Exp. Hematol., 17: 143-53, 1989) after irradiation of the adherent cells in long-term bone marrow culture). The murine fibroblast cell line MS-5 can be obtained, for instance, from the biological resource center DSMZ under reference n° ACC 441 (Braunschweig, Germany).

All references cited herein, including journal articles or abstracts, published patent applications, issued patents or any other references, are entirely incorporated by reference herein, including all data, tables, figures and text presented in the cited references.

The invention will be further evaluated in view of the following examples and figures.

### DESCRIPTION OF THE FIGURES

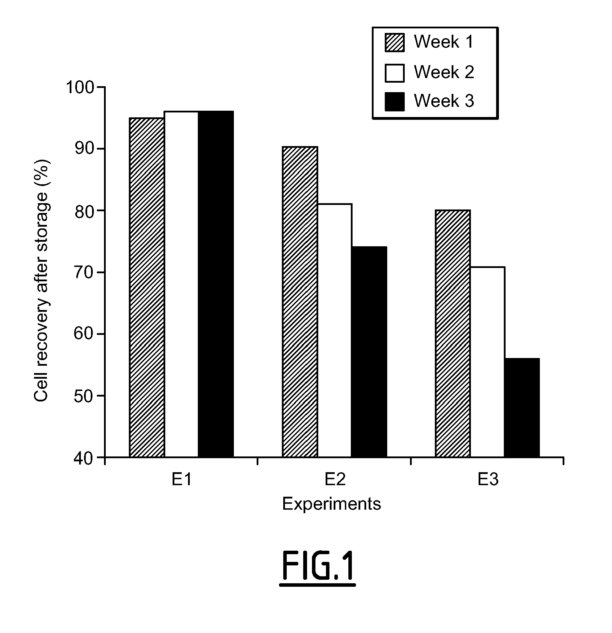

**Figure 1****:** Conservation capacity of cultured RBC as compared to native RBC.
   Three experiments are shown (E1, E2 and E3) to illustrate the variability of the cultured cells in terms of their conservation capacity. This figure illustrates the percentage of cell recovery after a given period of time of storage at 4°C. The x-axis represents the different experiments (each conducted during 1, 2 ad 3 weeks), and the y-axis represents the percentage of cell recovery after storage.
**Figure 2** illustrates the Kinetics of phosphatidylserine (PS) exposure during culture of cRBC. PS exposure was analyzed by monitoring the annexin-V-FITC binding of the erythroid cells at various stages of culture (lower right quadrant). Co-staining of the cells with propidium iodide (PI) allowed the detection of necrotic cells as annexin-V-positive/PI-positive cells (upper right quadrant). The percentage of enucleated cells (% cRBC) was mentioned above each corresponding dot plot.
**Figure 3** illustrates the results of calibration experiments regarding the optimal duration of the erythrophagocytosis (EP) test on MS-5 cells and human macrophages (MΦ). 3x10⁶ RBC (four and six weeks of age on MS-5 cells and MΦ, respectively) labeled with CFSE were incubated in 4 cm² wells for 1 to 4 h.
**Figure 4** illustrates the results of calibration experiments regarding the optimal number of RBC on MS-5 cells and human macrophages (MΦ). The dose effect of erythrocytes (10⁵, 10⁶, 3x10⁶ and 5x10⁶ RBC per 4 cm² well) was determined in tests in which the incubation time was fixed at 3 h.
**Figure 5** illustrates the effect of the dose of heat-denatured RBC (h-RBC) on erythrophagocytosis.
   EP tests using an increasing proportion of heat-treated native RBC (0 to 100%). The different mixtures were completed with untreated native RBC, e.g., the mixture denoted 10% h-RBC contained 10% heat-treated and 90% untreated RBC.
**Figure 6** illustrates the relationship between the phagocytic index and the cell yield after 3 weeks of storage for cRBC.
   Correlation between the phagocytic index and the cell yield after storage of cRBC. Scatter diagram comparing the phagocytic index of cRBC (x-axis) and their cell yield (y-axis, percentage recovery after 3 weeks of storage with respect to the number of cRBC at time t0 of storage). The phagocytic index was evaluated at time t0 of storage and data were analyzed with the Spearman test.

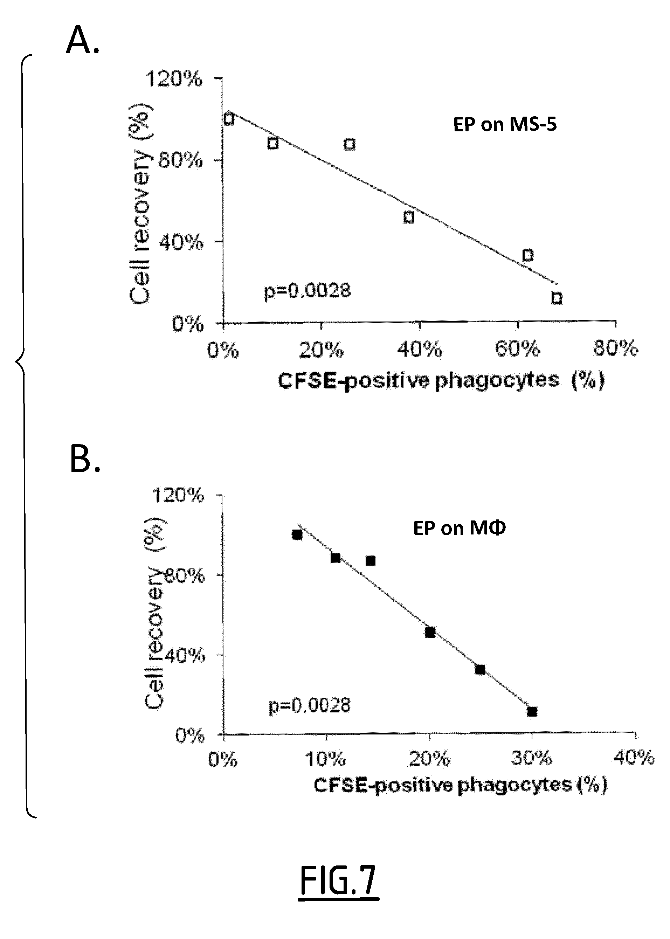

**Figure 7** illustrates the relationship between the phagocytic index and the cell yield after 3 weeks of storage for native RBC (B).
   Scatter diagram comparing the phagocytic index of heat-treated native RBC (x-axis) and their cell yield (y-axis, percentage recovery after 3 weeks of storage with respect to the number of RBC at time t0 of storage). Treated and untreated RBC were mixed in increasing ratios. The phagocytic index was evaluated at time t0 of storage and data were analyzed with the Spearman test.

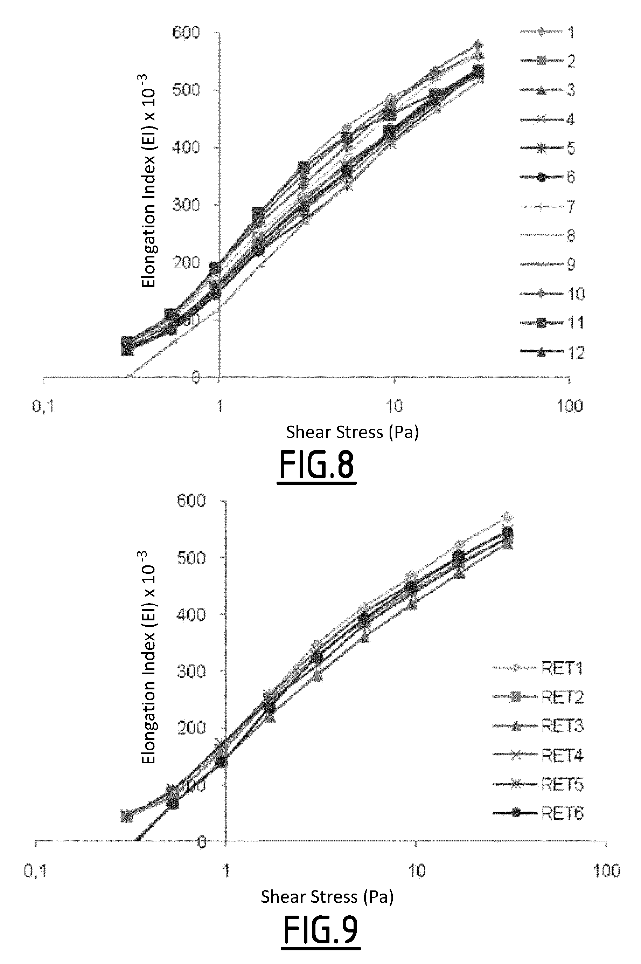

**Figure 8** illustrates the deformability profiles of cultured reticulocytes. The deformability (elongation index, EI) of cultured RBC (cRBC, n=12) reticulocytes was measured over a range of shear stresses (0.3-30 Pa) using the LORCA technique.
**Figure 9** illustrates the deformability profiles of native reticulocytes.
   The deformability (elongation index, EI) of native reticulocytes (RET, n=6) was measured over a range of shear stresses (0.3-30 Pa) using the LORCA technique.

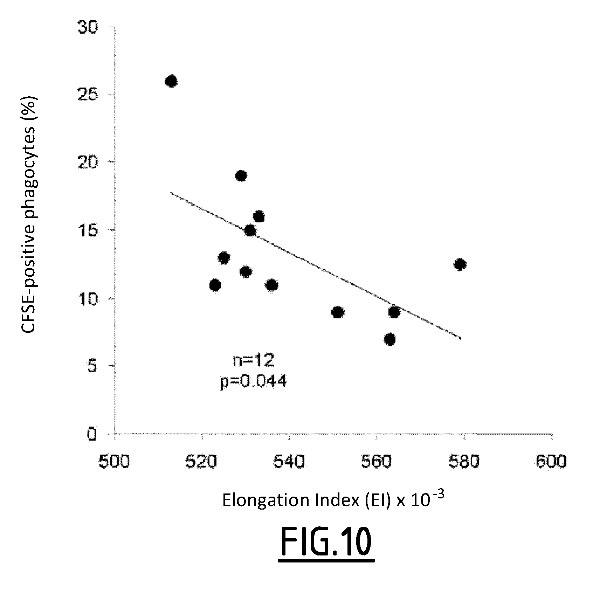

**Figure 10** illustrates the correlation between the phagocytic index and the elongation index of cRBC. Scatter diagram comparing the elongation index (Elmax) of cRBC (x-axis) and their phagocytic index (y-axis). Data were analyzed with the Spearman test.

### EXAMPLES

### Example 1:Materials and Methods

### 1.1. Sample preparation

### Controls

Blood samples freshly drawn into heparin from healthy donors (18-60 years old) constituted the negative control group. Blood samples were depleted of platelets by centrifugation (10 min at 200 x g) and washed in PBS (pH 7.4; Invitrogen). The red cells obtained were then depleted of leukocytes by centrifugation (10 min at 1300 x g) followed by careful removal with a pipette of the upper layer enriched in white cells. The cells were resuspended in PBS before use.

Aliquots from the same blood samples were kept without preservative solution at 4°C for 4 to 6 weeks were referred to as "aged RBC"(a-RBC) and constituted the positive controls. Aged RBC were washed twice in PBS by centrifugation (10 min at 200 x g) before use.

### Cultured red blood cells

The cRBC were generated according to the protocol previously described by Giarratana et al. (Blood, 118(19): 5071-9, 2011) from CD34+ cells derived from cord blood units (CB, n=18 from 13 healthy donors) or leukapheresis units obtained after stimulation with G-CSF (LK, n=20 from 9 donors) from healthy donors of hematopoietic stem cells. Briefly, the CD34+ cells were cultured in the presence of IMDM supplemented with human plasma and with sequential addition of SCF, IL-3 and Epo. The cells were fed twice a week with fresh medium. After 18 days, a majority of the cells were reticulocytes, as shown by their expression of CD71 (87 ± 4%) and by new methylene blue staining (89 ± 1%). The cell suspensions were purified by passage through a deleukocyting filter to eliminate the expelled nuclei and residual erythroblasts. The purity of the cRBC samples was 99.3 ± 0.2 % after filtration and the filtered suspensions were washed twice in PBS by centrifugation (10 min at 200 x g).

### Reticulocyte separation

Native reticulocytes were isolated from peripheral blood by an immunomagnetic method (Miltenyi Biotec). Briefly, the cells were incubated with anti-CD71 microbeads (Miltenyi) and the labeled cells were enriched on Mini-MACS columns. The reticulocyte content of the CD71-purified population was controlled by new methylene blue staining (> 90% of cells with intense staining) as previously described by Giarratana et al. (Blood, 118(19): 5071-9, 2011). Cells with at least 2 granules were scored as reticulocytes.

### Preparation of heat-denatured RBC

Fresh samples containing 10⁹ RBC depleted of platelets and leukocytes were denatured at 48°C for 60 min (referred to as heat-teated RBC, h-RBC). The suspensions were mixed every 15 min. The h-RBC were then cooled on crushed ice for 10 min and washed generously in PBS.

### Desialization of RBC by treatment with neuraminidase

Aliquots of 5x10⁷ RBC depleted of platelets and leukocytes were incubated in 100 µL of PBS containing 0.015 U of neuraminidase from Vibrio chlolerae (Sigma) for 1 h at 37°C under agitation (according to the protocol disposed by Bratosin et al., Cytometry, 30: 269-74, 1997). The cells were then washed three times in PBS.

### Opsonization of RBC

Aliquots of 5x10⁷ RBC derived from an Rh+ donor and depleted of platelets and leukocytes were incubated in 100 µL of PBS containing 5 µL of anti-RhD (Seraclone, Biotest AG, Dreieich, Germany; clone IgG BS221/H4111 B7; clone IgM BS232) for 1 h at 4°C and then washed once in PBS (according to the protocol disclosed by Fendel et al., Cytometry A, 71: 258-64, 2007).

### 1.2. Labeling of cells with CFSE

Red blood cells (RBC, cRBC) were labeled with carboxyfluorescein (CFSE, 5-6-carboxyfluorescein diacetate, succinimidyl ester; Interchim, Montluçon, France) as described by Fendel et al. (Cytometry A, 71: 258-64, 2007). Samples containing 10⁷ cells were incubated in a water bath at 37°C for 15 min h the presence of 5 µM CFSE. The labeled cells were generously washed three times at 4°C in PBS containing 5% fetal calf serum (FCS).

### 1.3. Phagocyte preparation

### Generation of human macrophages (abbreviated as "MΦ") from CD34+ bone marrow cells (reference method)

Human macrophages were produced by directed myeloid differentiation over 16 to 20 days of CD34+ cells isolated from bone marrow cells destined for an allograft. The CD34+ cells were separated by supermagnetic microbead selection using Mini-MACS columns (Miltenyi Biotec). Aliquots containing 10⁵ CD34+ cells/mL were cultured at 37°C under a 5% CO2 atmosphere in 24-well plates in IMDM-Glutamax (Biochrom, Berlin, Germany) supplemented with 20% FCS, SCF (50 ng/mL), FLT3-L (30 ng/mL), TPO (15 ng/mL) and IL3 (30 ng/mL). On day 5, the cells were adjusted to 10⁵/mL. On day 11, culture flasks were seeded with 2 x 10⁵ cells/cm2 in the presence of IMDM-Glutamax containing 20% FCS, SCF (25 ng/mL), IL3 (30 ng/mL) and M-CSF (30 ng/mL). Macrophages were obtained after 3 to 10 days of culture. On day 14, the adherent cells were harvested with trypsin and reseeded in 12-well plates at 1.5 x 10⁵ cells/cm² in IMDM-Glutamax supplemented with 20% FCS and M-CSF. The nature of the cells obtained was confirmed in each batch by the presence of the antigens CD14 and HLA-DR, expressed at 88 ± 4 % and 96 ± 1 %, respectively.

### Generation of phagocytes from the MS-5 cell line

The MS-5 cell line (Itoh et al. (Exp. Hematol., 17: 143-53, 1989), currently available from DSMZ (Braunschweig, Germany), was maintained in αMEM-Glutamax enriched in ribonucleosides and deoxyribonucleosides (Invitrogen, Paisley, Scotland) and supplemented with 10% FCS. At confluence, the adherent cells were harvested by treatment with trypsin-EDTA 1X for 7-10 min at 37°C The harvested cells (generally 106 cells/25 cm²) were washed and reseeded at 40,000 cells/cm² in αMEM-Glutamax containing 10% FCS in 12-well plates, each well corresponding to a surface area of 4 cm². The MS-5 cells were used 24 to 48 h after their passage and maintained at 37°C under 5% CO2.

### 1.4. Erythrophagocytosis (EP) test

### Incubation phase

The technique is derived from the work of Bratosin (Bratosin Det al., Cytometry, 30: 269-74, 1997). RBC (1 to 5 x 10⁶) labeled with CFSE were resuspended in 1 mL of IMDM supplemented with 20% FCS. The cells were added to adherent phagocytes previously prepared in 4 cm² wells and the mixture was incubated at 37°C under5% CO₂ for 1 to 4 h. The optimal quantity of target cells and the optimal duration of the test were determined using aged RBC (abbreviated as a-RBC). The sensitivity of the test was calculated from the dose effect of h-RBC.

Controls were incorporated into the EP test as follows: (i) for each sample, the autofluorescence of the phagocytic cells was determined after ingestion of non CFSE-labeled RBC/cRBC and (ii) fresh and aged labeled RBC from healthy donors were used as negative and positive controls, respectively.

### Collection of the phagocytes after incubation

The non phagocyted RBC were removed by washing carefully 3 to 4 times with cold PBS. The residual non phagocyted RBC were then hemolysed by addition of 0.9 mL of distilled water to each well (2 min) and isotonicity was re-established by adding 0.1 mL of PBS 10X. Macrophages were collected by scraping and MS-5 cells by rapid treatment with trypsin (5-6 min). The cells were washed generously in PBS (pH 7.4). MS-5 cells were resuspended in 300 µL of PBS containing 2% FCS for direct flow cytometric analyses.

### 1.5. Flow cytometric analyses

### Phagocytosis

The human macrophages collected after the erythrophagocytosis test were labeled with a phycoerythrin (PE)-conjugated monoclonal antibody not recognizing erythrocytes (e.g. CD14, HLA-DR, CD45....). Samples were labeled with the murine anti-human antibodies (Beckman Coulter) at ambient temperature for 15 min and then washed and isotype matched PE-conjugated antibodies were used as controls. The cells were resuspended in 300 µL of PBS containing 2% FCS.

The macrophagic cells (human or murine) were analyzed in a FacScalibur flow cytometer (BD Biosciences) equipped with a 488 nm laser and the data were integrated with Cellquest software (BD). A total of 10,000 events were recorded in the "size/structure" system, where one determines a gate of interest R1 which excludes cell debris. The CFSE signal was analyzed when the co-culture was performed on MS-5 cells and the double signal CFSE/HLA-DR-PE when the co-culture was performed on human macrophages. The percentages of CFSE⁺ and CFSE⁺/HLA-DR-PE⁺ cells are indicated in the corresponding quadrants.

### Detection of phosphatidylserine with annexin V-FITC

According to the recommendations of the manufacturer (Beckman Coulter), 5 x 10⁵ RBC were incubated in 100 µL of cold buffer (pH 7.4) containing CaCl₂, 1 µL of annexin V-FITC (fluorescein isothiocyanate) and 5 µL of propidium iodide (PI) for 15 min on ice. The incubation was terminated by adding 300 µL of cold buffer and the cells were analyzed immediately. A control without annexin V or PI was performed under the same conditions.

### Measurement of the expression of CD47 at the surface of RBC and cRBC

The cells were washed in PBS (pH 7.4) and adjusted to 10⁶/mL in the presence of 2% FCS. Aliquots of 10⁵ cells were incubated with 10 µL of anti-CD47-FITC antibody (clone B6H12; BD Pharmingen) or an irrelevant isotype control for 20 min at ambient temperature, after which the cells were washed and resuspended in PBS containing FCS.

### Calcein AM viability test

The test was performed according to the procedure of Bratosin et al. (Cytometry A, 66: 78-84, 2005). Briefly, 2 x 10⁵ RBC were incubated in the presence of 5 µM calcein AM (Sigma, St Quentin Fallavier, France) in a final volume of 200 µL for 45 min at 37°C under aerobiosis. The cells were then resuspended in 500 µL of PBS (pH 7.4) and analyzed immediately. A control without calcein AM was performed under the same conditions. A cell was considered to be viable if the intensity of the fluorescence was superior to 10² on an arbitrary scale ranging from 1 to 10⁴.

### 1.6. Storage of the samples

RBC/cRBC (5 x 10⁷) were resuspended at 10⁷ cells/mL in Sag-Mannitol-based solution (saline-adenine-glucose-mannitol medium; Haemonetics) at 4°C and stored in a 5 mL glass tube for 1 to 3 weeks. To establish the cell yield with respect to time to for the storage of RBC/cRBC, the cells were counted weekly in a hemocytometer. The cell concentration of the stored sample was then calculated and compared to that of the non stored sample.

### 1.6. Deformability measurements

The cell flexibility of cRBC and native reticulocytes was determined using a laser diffraction technique [LORCA (Laser-assisted Optical Rotational Cell Analyzer); R&R Mechanotrics, Hoor, The Netherlands] as extensively described previously (Giarratana et al. (Blood, 118(19): 5071-9, 2011); Hardeman and Ince, Clin. Hemorheol. Microcirc., 21: 277-84, 1999). The cell deformability was expressed in terms of the elongation index (EI) which was recorded continuously at various shear stresses in the range 0.3-30 Pa. The EI value at 30 Pa was referred to as Elₘₐₓ.

### 1.7. Statistical tests

Correlations were established with the Spearman test and comparisons between different groups with the Man-Whitney U test. The difference was considered to be significant for a p value of < 0.05.

### 1.8. Microscopic analyses

### Optical microscopy

Cells were washed and spun onto slides by cytocentrifugation. Cytological examinations were performed on slides stained with May-Grunwald Giemsa (Sigma).

### Immunofluorescence microscopy

Immunofluorescence samples were processed as follows: the human macrophages collected after EP tests were washed and labeled in test tubes with a PE-conjugated anti-HLA-DR antibody. No further labeling was performed after EP tests using the MS-5 cell line. Aliquots of 3-4x10⁴ cells were washed and spun onto microscope slides by cytocentrifugation at 700 rpm for 3 min (Thermofisher). The immunofluorescence was assessed in a LEICA apparatus equipped with appropriate filters for the detection of CFSE- and PE-labeled cells.

### Example 2:Results

### 2.1. Storage of cRBC

Assessing the aptitude of cRBC for long-term storage, the inventors have noticed that capacity of cRBC for being stored for a long period of time varies from one batch to another. The cell yield of cRBC after 3 weeks of storage varied from one batch to another and ranged from 56 to 96%, with a mean of 82 ± 3% and a median of 85% (Fig. 1). No difference was observed between CB and LK CD34+ -derived cRBC in terms of the storage performance (80 ± 3% and 82 ± 3% at the third week, respectively).

In the face of the disparity of the batches of cRBC in terms of their capacity to resist to long-term storage, the inventors looked for parameters which might support this observation. In the literature the quality of RBC is classically evaluated according to certain parameters considered to be critical, namely the cell viability (determined by the calcein AM test), the expression of CD47 (antiphagocytosis receptor) and the externalization of phosphatidylserine (marker of phagocytosis).

Thus, the inventors decided to study these parameters.

The study showed that:
(i) The mean fluorescence intensity (MFI) of the calcein dye was dramatically increased in cRBC as compared to native RBC (see below Table 1, column 2), with MFI values of 2441 ± 110 (median 2443) and 1332 ± 50 (median 1359), respectively.

As cRBC are young cells, they exhibited an MFI pattern similar to that of native reticulocytes (see below Table 2, column 1). Although the intensity of the fluorescence was higher in cRBC as compared to native RBC, non viable cells (with an MFI of < 10²) were detected in most of the cultures, representing 1 to 14% of the overall population (median 4%, n=38; see below Table 1, column 1).

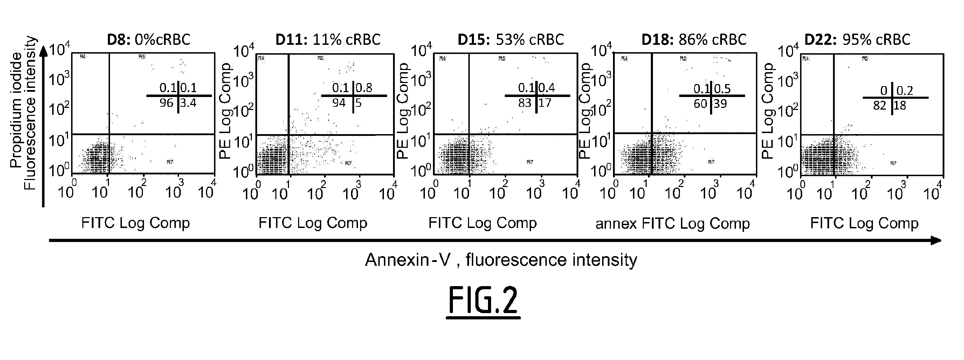

The recovery of cRBC after storage was nevertheless not correlated with either the percentage of viable cells or the MFI of the calcein dye, despite the range observed from one batch of cRBC to another (1345-3969). It is noteworthy that the disparity of the results in terms of MFI was similar in cRBC (1345-3969) and native reticulocytes (1502-3200) (see below Table 2).
(ii) cRBC readily bound annexin-V as compared to native RBC (see below Table 1, columns 3-4). Thus, the cRBC produced were mainly reticulocytes and consequently behaved like their native homologues with regard to the externalization of PS (29 ± 3% of native reticulocytes were annexin positive) (see below Table 2). Figure 2 shows the kinetics of the binding of annexin-V to erythroid cells during culture. The percentage of annexin-positive cells increased with terminal cell maturation, but without any increase in the proportion of necrotic cells since the percentage of annexin-positve/PI positive cells remained constantly low throughout culture. PS expression peaked on day 18 of culture (the day on which the cRBC were harvested) and then decreased during the maturation of the reticulocytes into mature cells when the culture was prolonged for 4 days (day 22, see Fig. 2). These results indicate that the PS exposure of the cRBC was a mark of their young age.

Consequently, no relationship between the proportion of cells expressing PS in the cRBC cultures and the cRBC recovery after long-term storage was observed, despite the variability among the batches (18-57%).
(iii) Concerning the level of expression of CD47, no significant difference was observed between cRBC and native RBC (see below Table 1, column 5), with MFI values of 101 ± 8 (median 91) and 107 ± 4 (median 106), respectively. There was no correlation between CD47 expression and the cRBC recovery after long-term storage.

**Table 1: Characteristics of native and cultured RBC (cRBC)**

| | **Calcein + cells (%)** | **Calcein⁺ cells (MFI*)** | **Annexin +cells (%)** | **Annexin⁺c ells (MFI)** | **CD47^{**} (MFI)** | **EP assay on MΦ: CFSE⁺ cells (%)** | **EP assay on MS-5: CFSE⁺ cells (%)** |
|---|---|---|---|---|---|---|---|
| **Native RBC** | | | | | | | |
| mean±SD | 99.5 ± 0.1 | 1332 ± 50 | 1.0 ± 0.1 | 25 ± 3 | 107 ± 4 | 5.3 ± 0.7 | 3.0 ± 0.4 |
| median | 99.8 | 1359 | 1.0 | 24 | 106 | 5 | 2.5 |
| [range] | [99- 100] | [1120-1430] | [0.5-1.3] | [11-55] | [86-135] | [2-11] | [1-16] |
| (n) | (n=9) | (n=22) | (n=16) | (n=16) | (n=15) | (n=16) | (n=26) |

| **cRBC** | | | | | | | |
|---|---|---|---|---|---|---|---|
| mean±SD | 96 ± 0.7 | 2441 ± 110 | 40 ± 3 | 31 ± 2 | 101 ± 8 | 11.6 ± 2.6 | 15.2 ± 2.6 |
| median | 96 | 2443 | 43 | 33 | 91 | 10 | 12 |
| [range] | [86- 100] | [1345-3969] | [18-57] | [20-45] | [71-176] | [3-34] | [4-47] |
| (n) | (n=38) | (n=26) | (n=19) | (n=19) | (n=16) | (n=11) | (n=38) |
| P*** | 0.0007 | <0.0001 | <0.0001 | 0.060 | NS | 0.015 | <0.0001 |

| | | | | | | | |
|---|---|---|---|---|---|---|---|
| * MFI: mean fluorescence intensity. ** All the samples were 100% positive for CD47 expression. *** Statistical comparisons evaluated with the Man Whitney U test. NS: non significant | | | | | | | |

**Table 2: Characteristics of native reticulocytes and cultured RBC (cRBC)**

| | **Calcein⁺ cells (MFI^{*})** | **Annexin⁺cells (%)** | **Annexin⁺cells (MFI)** |
|---|---|---|---|
| **Native reticulocytes** | | | |
| mean±SD | 2203 ± 197 | 29 ± 3 | 29 ± 2 |
| median | 2153 | 28 | 28 |
| [range] | [1502-3200] | [12-47] | [20-45] |
| (n) | (n=9) | (n=13) | (n=13) |

| **cRBC** | | | |
|---|---|---|---|
| mean±SD | 2441 ± 110 | 40 ± 3 | 31 ± 2 |
| median | 2443 | 43 | 33 |
| [range] | [1345-3969] | [18-57] | [20-45] |
| (n) | (n=38) | (n=19) | (n=19) |
| P** | NS | 0.016 | NS |

| | | | |
|---|---|---|---|
| * MFI: mean fluorescence intensity ** Statistical comparisons established with the Man Whitney U test. NS: non significant | | | |

### 2.2. Erythrophagocytosis test

Overall, neither the cell viability nor the expression of CD47 or the externalization of PS allowed qualitative discrimination of the batches of cRBC, or supported the observations concerning their aptitude for long-term storage.

These results led the inventors to search for another parameter that would correlate with the aptitude of cRBC to be stored for a long period of time. They decided to compare the batches of cRBC on the basis of their recognition by phagocytes.

### 2.2.1. Calibration of the erythrophagocytosis test

The test was calibrated to determine (i) the optimal duration of erythrophagocytosis, (ii) the optimal number of erythrocytes to incubate with the phagocytes and (iii) the sensitivity of the test after optimization of the conditions.

This procedure was necessary because each type of phagocyte has its own characteristics. The EP tests were performed comparatively using the murine fibroblast cell line MS-5 and as the reference technique human macrophages (MΦ).

### Duration of incubation and optimal quantity of target cells

Preliminary experiments showed that aged RBC (a-RBC), produced by 4-6 weeks storage of heparinized whole blood, were strongly recognized by both the MS-5 cell line and human macrophages. The inventors took advantage of this fact to calibrate the erythrophagocytosis assay.

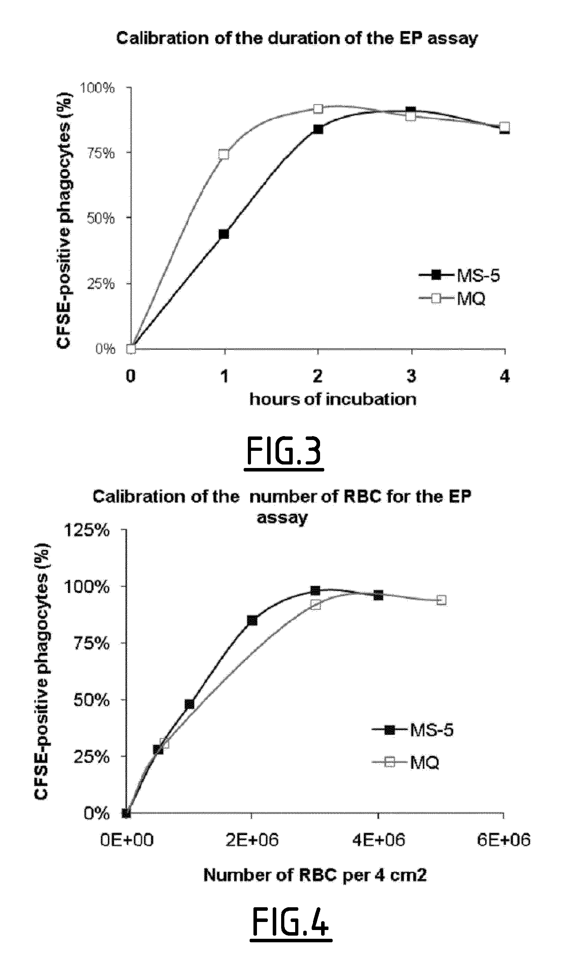

A plateau response was observed at 2 h for human macrophages and 3 h for the MS-5 cell line and hence a duration of 3 h was retained for all subsequent assays (Fig. 3). The dose effect of RBC labeled with CFSE was measured using an incubation time of 3 h. A plateau response was obtained for 3x10⁶ cells per 4 cm² well, whatever the phagocytes employed (Fig. 4). Thus, the optimal number of RBC was about 8x10⁵ cells/cm², i.e., a ratio of 20 to 40 cells per phagocyte.

In summary, the conditions retained for the incubation time and quantity of RBC were respectively: 3 h and 3x10⁶ RBC per 4 cm² well.

### Sensitivity and linearity of the erythrophagocytosis test

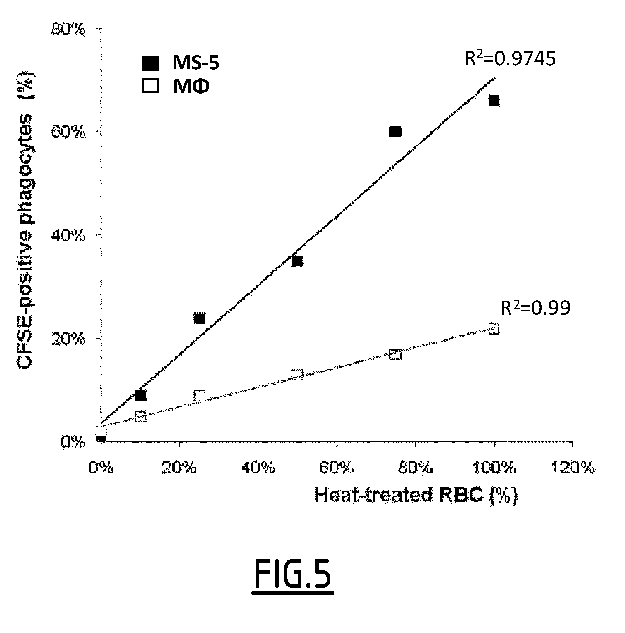

These parameters were determined from the dose effect of heat-denatured RBC (h-RBC), the thermal treatment inducing principally the denaturation of spectrin ³. Treated and untreated RBC were mixed in increasing ratios. It was found that for the two types of phagocyte the intensity of erythrophagocytosis (abbreviated as "EP") was proportional to the quantity of denatured RBC (Fig. 5). In addition, the MS-5 cell line was clearly more sensitive than MΦ.

These results showed that the two types of phagocyte recognized desialized erythrocytes. Opsonized RBC were nevertheless not recognized by MS-5 cells since the latter lack Fc sites (see below Table 3). In contrast, the response of MS-5 cells increased dramatically as a function of the storage time of a-RBC (see below Table 4, lines 1 and 2). The extent of the damage to "erythrocytes aged *in vitro*" was assessed by the binding of annexin V (see below Table 4, line 3). If very aged blood samples gave similar information using the two types of phagocyte, little or moderately aged samples were only weakly recognized by MΦ as compared to the MS-5 line.

**Table 3: Comparative erythrophagocytosis assay with treated RBC***

| | **Native RBC** | **Neuraminidase- treated RBC** | **Opsonized RBC** |
|---|---|---|---|
| EP on MS-5 (n=3) | 3.5 ± 1.5 | 11.2 ± 9.1 | 2.1 ± 1 |
| (range) | (2 - 4.8) | (5 - 22) | (1.4 - 3.2) |
| EP on MΦ (n=3) | 4.3 ± 1.1 | 16.3 ± 9.7 | 21 ± 8.3 |
| (range) | (3 - 4.9) | (8 - 27) | (11 - 27) |

Results are expressed in terms of the percentage of CFSE+ cells (mean ± SEM).

**Table 4: Erythrophagocytosis assay and PS externalization for increasingly aged RBC**

| | **Week 0** | **Week 1** | **Week 2** | **Week 3** | **Week 4** | **Week 5** | **Week 6** |
|---|---|---|---|---|---|---|---|
| EP^{(a)} on MS-5 | 3 ± 0.4 | 15 ± 11 | 59 ± 25 | 88 ± 10 | 92 ± 9 | 90 ± 10 | 97 ± 2 |
| | (n=26) | (n=4) | (n=6) | (n=12) | (n=16) | (n=3) | (n=5) |
| EP^{(a)} on MΦ | 5 ± 0.7 | 5 ± 2 | 7 ± 2 | 19 ± 14 | 44 ± 25 | 60 ± 28 | 77 ± 16 |
| | (n=16) | (n=3) | (n=4) | (n=4) | (n=5) | (n=3) | (n=5) |
| Annexin⁺ cells (%) | 2.1 ± 0.3 | 7 ± 6 | 15 ± 9 | 36 ± 5 | 40 ± 19 | nd | nd |
| | (n=12) | (n=4) | (n=9) | (n=3) | (n=5) | | |

| | | | | | | | |
|---|---|---|---|---|---|---|---|
| (a) Results are expressed in terms of the percentage of CFSE+ cells. The number of experiments is indicated in brackets. Mean ± SEM. | | | | | | | |

### 2.2.2. Erythrophagocytosis assay

The EP assays on MS-5 cells and MΦ were able to reveal damaged cells, as demonstrated using aged RBC (See Table 4), desialized cells (see Table 3) and an increasing ratio of heat-denatured RBC (Fig. 5). Taking advantage of these observations, EP tests were performed with cRBC and compared them to native RBC. In addition, the lack of Fc sites on the MS-5 cell line enabled us to establish that the EP test results were not linked to any opsonization phenomenon.

Native RBC from healthy donors were as expected only weakly recognized by only a small proportion of the phagocytes, whatever their origin (see above Table 1, columns 6-7). Thus, the mean phagocytic index was 5.3 ± 0.7% (2 to 11%, n=16) and 3.0 ± 0.4% (1 to 16%, n=26), respectively, for human macrophages and MS-5 cells.

The overall phagocytic index of cRBC was significantly higher than that of native RBC (see above Table 1). The median EP index was 10% but ranged from 3 to 34% using MΦ (n=11) and from 4 to 47% using MS-5 cells (n=38). No significant difference in phagocytic index was observed between cRBC derived from cord blood cells (CB) or from G-CSF-stimulated LK cells (LK) (mean 17 ± 3% and median 14% for CB, n=18; mean 12 ± 2% and median 11 % for LK, n=20).

### 2.2.3. Relationship between Erythrophagocytosis and capacity of cRBC to be stored

The wide ranges of results for the EP assays and cRBC yield during storage prompted the inventors to search for a relationship between these two parameters.

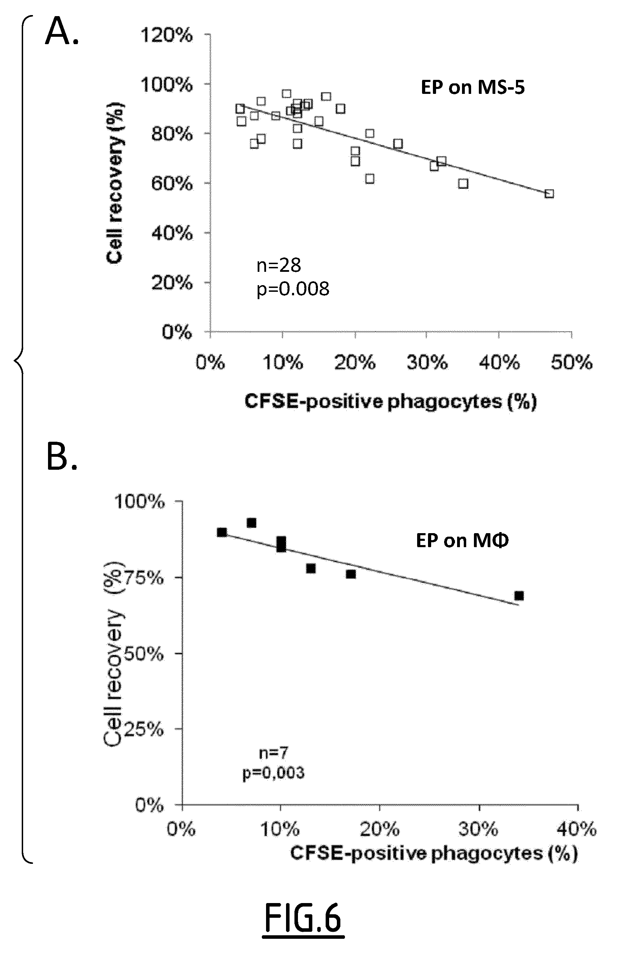

Figure 6 depicts the relationship between the cell yield of cRBC after 3 weeks of storage and the phagocytic index at time t0 of the conservation test (p=0.008 and p=0.003, respectively, for MS-5 cells (see Figure 6A) and MΦ (see Figure 6B)). These observations were confirmed using heat-denatured native RBC (Fig. 7A and 7B). Among the 28 batches of cRBC studied, 7/28 (25%) had a cell yield of less than 75% after 3 weeks of storage and all 7 of these batches had a higher phagocytic index compared to the maximum level observed for native RBC.

### 2.3. Deformability of cRBC

To determine whether the EP and storage test results were linked to the presence of damaged cells, the deformability of 12 batches of cRBC was tested using the LORCA technique (Fig. 8). The value of Elmax ranged from 0.513 to 0.579 for cRBC. It was noted that the cRBC previously injected into human displayed an Elmax of 0.528 (see Giarratana et al., Blood, 118(19): 5071-9, 2011). Since the deformability profile is related to the status of the cells, cRBC were compared to native reticulocytes. The Elmax of the latter ranged from 0.525 to 0.571 (Fig. 9). These results showed that the phagocytic index of the cRBC was inversely correlated with the value of Elmax (p=0.044) (Fig. 10).

### Example 3:Conclusions

The present study highlighted the batch-to-batch heterogeneity of cRBC. These disparities were not linked to the origin of the cells, i.e., cord blood versus leukapheresis cells. On the contrary, variations of the phagocytic index between batches of cells generated from samples derived from the same donor were observed. Interestingly, the cell yield after 3 weeks of storage was low in the samples exhibiting a high phagocytic index. These findings showed that the observed dispersity of the phagocytic index is not donor dependent but culture condition dependent.

No direct evidence for the presence of damaged erythrocytes in the *in vitro* cell cultures could be formally confirmed through the monitoring of PS expression or calcein dye staining, while the CD47 protein was not recognized in our xenogenic EP assay, excluding its involvement in erythrophagocytosis. Furthermore, the levels of expression of calcein dye and PS reflected more the young age of the cells rather than signs of cell damage.

However, a relationship exists between the cell yield after 3 weeks of storage and the phagocytic index at time t0 of the conservation test, starting from either cRBC or heat-denatured native RBC, both of which displayed obvious hemolysis during storage.

The difficulties in controlling all the cell culture parameters may be responsible for the generation of damaged cells. The phagocytosis of cRBC could thus be the consequence of membrane alterations induced by culture and the results of ektacytometry measurements supported this hypothesis.

In establishing a relationship between the deformability index and the phagocytic index, the inventors show that the EP test may be used as an alternative assay for the global evaluation of cRBC, recalling that the access to ektacytometer is more restricted than that to cell culture.

Further, when performing the erythrophagocytosis assay, the inventors showed that the MS-5 line displays a sensitivity superior to that of MΦ, notably with regard to erythrocytes "aged in vitro" or RBC presenting a modification of the cytoskeleton (induced by heating *in vitro*).

## Claims

1. A method for assessing the quality of a batch of red blood cells generated *in vitro* (cRBCs), said method comprises the following steps:
a) bringing into contact with phagocytes a sample of cRBCs from the batch to be tested;
b) determining the percentage of the cRBCs which is phagocytosed (erythrophagocytosis quantification);
c) optionally, deducing from the result of step b) the quality of the batch of cRBCs.

2. A method for evaluating the capacity of a batch of red blood cells generated *in vitro* (cRBCs) to be stored for a long period of time, said method comprises the following steps:
a) bringing into contact with phagocytes a sample of cRBCs from the batch to be tested;
b) determining the percentage of the cRBCs which are phagocytosed (erythrophagocytosis quantification);
c) optionally, deducing from the result of step b) the capacity of said batch of cRBCs to be stored for a long period of time.

3. The method according to claim 1 or 2, wherein before step (b) is a step (a') consisting of removing the non-phagocytosed cRBCs.

4. The method according to any one of claims 1 to 3, wherein the cRBCs of step a) are stained with a marker chosen from fluorochrome markers and radioactive markers.

5. The method according to any one of claims 1 to 3, wherein the cRBCs of step a) are stained with a biological dye.

6. The method according to any one of claims 1 to 5, wherein the ratio [number of cRBCs / number of phagocytes] in step a) is from 10/1 to 1/80.

7. The method according to claim 6, wherein the ratio [number of cRBCs / number of phagocytes] in step a) is from 20/1 to 40/1.

8. The method according to any one of claims 1 to 7, wherein step (a) is carried out for 1 to 5 hours.

9. A batch of cRBCs of good quality and/or suitable for long-term storage obtainable by the methods as defined in any one of claims 1 to 8.

10. A kit for assessing the quality of a batch of cRBCs, and/or for evaluating the capacity of said batch to be stored for a long period of time, which kit comprises the following means:
i) phagocytes;
ii) at least one marker chosen from fluorochrome markers and radioactive markers, or at least one biological dye;
iii) a positive control sample consisting of red blood cells, the red blood cells of which would be phagocytosed if they were contacted with phagocytes.

11. The kit according to claim 10, wherein the red blood cells (iii) are chosen from the group consisting of:
(a) aged red blood cells;
(b) heat-denatured red blood cells;
(c) desialized-red blood cells;
(d) red blood cells treated with glutaraldehyde.

12. The kit according to claim 10 or 11, wherein the red blood cells of (iii) are native red blood cells or red blood cells generated *in vitro.*

13. The kit according to claim 10, wherein the red blood cells (iii) are provided in glutaraldehyde solution or in glutaraldehyde / formaldehyde mix solution.

14. The kit according to any one of claims 10 to 13, wherein the phagocytes (i) are the murine fibroblast cell line MS-5.

15. The kit according to any one of claims 10 to 14, wherein the red blood cells (iii) are labeled with the same marker as marker (ii) of the kit, or are stained with the same dye as dye (ii) of the kit.
